# EUROPEAN PATENT SPECIFICATION

(11) **EP 1 392 229 B1**
(45) Date of publication and mention of the grant of the patent: **24.01.2007**
(21) Application number: 02733586.8
(22) Date of filing: 09.04.2002
(51) Int. Cl.: A61Q 11/00, A61K 8/24

(54) **REMINERALIZING DENTAL HYGIENE PRODUCTS**
REMINERALISIERENDE ZAHNHYGIENEPRODUKTE
PRODUITS D'HYGIENE DENTAIRE REMINERALISANTS

(30) Priority: 05.06.2001 NL 1018208
(43) Date of publication of application: 03.03.2004
(73) Proprietor: Calcio B.V., 6109 AH Ohé en Laak (NL)
(72) Inventor: DRIESSENS, Ferdinand, Clemens, Maria, NL-6109 AH Ohé en Laak (NL); BOLTONG, Maria, Gertruda, NL-6109 AH Ohé en Laak (NL)
(74) Representative: Valkonet, Rutger
(86) International application number: PCT/NL2002/000265
(87) International publication number: WO 2002/098383

(56) References cited:
- EP-A- 0 543 765
- WO-A-01/12242
- DE-U- 29 914 313
- US-A- 5 262 166

## Description

The present invention relates to remineralizing dental hygiene products like tooth pastes, chewing gums, mouth rinsing fluids, saliva substitutes and gel suspensions.

Toothpastes have been known for a long time, for example from US patents nos. 5,605,677 and 5,993,786. From the former US patent, for example, a polishing agent is known which comprises silica and dicalcium phosphate dihydrate in a ratio by weight of 10:1 - 1:1. One drawback of such a formulation is that in a slightly alkaline environment, as can be found in the mouth of a user, phosphate and calcium ions are released to such an extent that it leads to precipitation of calcium phosphate. Thus, such formulations will enhance the development of dental calculus which normally occurs in a slightly alkaline environment, in particular in the user's mouth.

At present many dental hygiene products are fluoridated. The action of fluoride in oral affairs is that it retards the demineralization of tooth enamel which is due to plaque activity, and so helps to prevent the growth of initial carious lesions. There is no proof whatsoever that these products lead to remineralization of carious lesions (see e.g. F.C.M. Driessens, Mineral Aspects of Dentistry, S. Karger, Basel, 1982 and F.C.M. Driessens and R.M.H. Verbeeck, Biominerals, CRC Press, Boca Raton, 1990). Neither do these products lead to desensitization of dentine lesions in the tooth neck region. Therefore, there is a big need for remineralizing dental hygiene products.

Up to now, the only evidence that under natural conditions remineralization of initial carious lesions can occur, comes from epidemiological studies (0. Backer Dirks, Longitudinal dental caries study in children 5-15 years of age, Arch. Oral Biol. 6 (1961) 94-121). There is agreement among dental researchers about the fact that the remineralizing power is located in the saliva, but despite extensive studies it is not known which degree of saturation or supersaturation in the saliva is reponsible for this remineralizing power, be it that of brushite, that of monetite, that of octacalcium phosphate, that of calcium deficient hydroxyapatite, or that of hydroxyapatite.

It is an object of the present invention to provide dental hygiene products in the form of tooth pastes, chewing gums, mouth rinsing fluids, saliva substitutes and gel suspensions, which have remineralizing power in the natural oral environment, even under slightly acidic conditions where otherwise carious lesions would be formed.

Another object of the present invention is to provide dental hygiene products in the form of tooth pastes, chewing gums, mouth rinsing fluids, saliva substitutes and gel suspensions which slow down the demineralization of tooth tissues caused by dental plaque activity.

A further object of the present invention is to provide dental hygiene products in the form of tooth pastes, chewing gums, mouth rinsing fluids, saliva substitutes and gel suspensions which counteract the demineralization of tooth enamel and dentin caused by therapeutic etching fluids applied during dental treatments.

The present invention is characterized in that the remineralizing hygiene products comprise CaKPO₄ and/or Ca₂KNa(PO₄)₂.

The CaKPO₄ and Ca₂NaK(PO₄)₂ are solid substances, which exhibit a relatively high solubility and which are furthermore stable in a slightly alkaline environment. When a heavily acidic solution in the form of acetic acid or lactic acid is added thereto, however, a reaction will take place so as to form a precipitate of potassium containing apatite (K-A.) Said K-A releases some calcium and phosphate ions but only in a relatively low concentration. Better for remineralization is a slightly acidic environment which will fully use the remineralizing power of these compounds. In practice the slight acidification of the dental hygiene product, which comprises CaKPO₄ and/or Ca₂KNa(PO₄)₂, will be caused by the slightly acidic compounds that are present in the mouth when caries occurs therein. Thus, the remineralizing activity of the present dental hygiene product, which comprises CaKPO₄ and/or Ca₂KNa(PO₄)₂, will take place automatically due to the slightly acidic environment in the mouth.

During the last 15 years we have worked on the development of calcium phosphate cements. Up to now the commercial calcium phosphate cements are apatitic in nature. However, there are three types. See Table 1.

**Table 1.**

| Prototype formula | Abbreviation | pH range |
|---|---|---|
| Ca₁₀(PO₄)₆(OH)₂ | HA | 10 < pH < 11.5 |
| Ca₉(HPO₄)(PO₄)₅OH | CDHA | 8 < pH < 9.5 |
| Ca₅K₄₋ₓNaₓ(HPO₄)₄(PO₄)₂(H₂O) | K-A | 6 < pH < 7.5 |

HA is hydroxyapatite, CDHA is calcium deficient hydroxyapatite and K-A is potassium containing apatite. HA and CDHA can also contain some Na and CO₃. The pH ranges in this table are very important. They mean that the corresponding cement powders react preferably to form the corresponding apatites in the indicated pH range. Reversely, it means that the indicated apatites form by precipitation only within the corresponding pH ranges. As the pH of human saliva is in the range 5.5 < pH < 7.5, the only apatite which can precipitate from human saliva is potassium containing apatite K-A. Therefore, we come to the conclusion that the remineralizing power of saliva is located in its supersaturating with K-A. And, indeed, human saliva contains considerable amounts of calcium ions, phosphate ions, potassium ions (and sodium ions) (F.C.H. Driessens, Mineral Aspects of Dentistry, S. Karger, Basel, 1982).

This also means that on the basis of this knowledge we can design remineralizing dental hygiene products which have even higher remineralizing power than the natural saliva. Important for that purpose is the use of the compounds which are parent for the formation of K-A cements. These are CaKPO₄ and/or Ca₂KNa(PO₄)₂. These have a relatively high solubility compared to e.g. brushite, monetite, octacalcium pohosphate, calcium deficient hydroxyapatite and hydroxyapatite, and, in addition, they contain all the ingredients necessary for the formation of K-A precipitates. Therefore, tooth pastes, chewing gums and gel suspensions with remineralizing power can be prepared by designing formulations which comprise CaKPO₄ and/or Ca₂KNa(PO₄)₂ and which are adjusted to a suitable pH. Moreover, mouth rinsing fluids and saliva substitutes with remineralizing power can be prepared by designing formulations which comprise dissolved calcium ions, potassium ions and phosphate ions from whatever suitable sources are available and in such a way that these products are adjusted to a suitable pH.

The suitability of the pH in these products is determined by a value at which the solubility of CaKPO₄ and/or Ca₂KNa(PO₄)₂ is as high as possible without the occurrence of spontaneous nucleation of potassium containing apatite K-A. In our research on calcium phosphate cements we have noticed that spontaneous nucleation of K-A occurs, when CaKPO₄ crystals and/or Ca₂KNa(PO₄)₂ crystals are in contact with aqueous solutions having a pH below 4. Hence, pH values suitable for the remineralizing dental hygiene products, which we aim at, is between pH 4 and pH 8, preferably between pH 5 and pH 7. The reason is that products at a pH below 4 will loose most of their remineralizing capacity too soon by spontaneous nucleation of K-A and will therefore have a shelf life which is too short, whereas the remineralizing power of these products will be too low when their pH is higher than 8 due to the limited solubility of CaKPO₄ and/or Ca₂KNa(PO₄)₂ at such pH values. Hence, we aim at pH values whereby K-A apatite can have crystal growth on existing apatite crystals, but whereby spontaneous K-A nucleation does not occur. Thereby, the existing apatite crystals are meant to be the HA crystals in tooth enamel and the CDHA crystals of dentine. As known from our in vitro work, the K-A layers which grow on the HA and CDHA crystals are spontaneously transformed into HA and CDHA respectively by maturation. In this way, the crystals in initial carious lesions in tooth enamel and dentine can be healed by ongrowth first of K-A layers which are transformed into the original HA and CDHA apatite by spontaneous maturation later on.

It is important to notice that in the remineralizing dental hygiene products of the present invention the amounts of fluoride or zinc should be as low as possible. Small amounts of fluoride will destroy the remineralizing power by precipitation of calcium fluoride, whereas zinc will do the same by precipitation of zinc phosphate.

In the remineralizing dental hygiene products according to the present invention the amount of CaKPO₄ and/or Ca₂KNa(PO₄)₂ preferably ranges between 1 and 80 wt.%, especially between 5 and 70 wt.%, in particular between 20 and 50 wt.%, based on the total weight of the products. As far as tooth pastes are concerned it should be understood that they also may comprise other polishing agents which are usual according to the prior art, such as silica, for example. These products may also comprise one or more of the components from the group consisting of water, thickeners, foaming agents, lubricants, enzymes, colorants, flavourings and moisturizers as additives. If the amount of CaKPO₄ and/or Ca₂KNa(PO₄)₂ is less than 1 wt.%, the remineralizing capacity of these products will be too small. On the other hand, an amount of more than 80 wt.% will lead to products which are unmanageable, which are brittle and crumbling, and which will desintegrate.

The mouth rinsing fluids and the saliva substitutes of the present invention comprise an aqueous solution containing calcium ions, phosphate ions and potassium ions in such a way that their concentrations are higher than those of equilibrium with K-A at the adjusted pH value, but equal to or lower than saturation with CaKPO₄ and/or Ca₂KNa(PO₄)₂ at the adjusted pH. Additional ions may comprise sodium and chloride for example. Furthermore, these dental hygiene products may comprise one or more of the components from the group consisting of water, thickeners, foaming agents, lubricants, enzymes, colorants, flavourings and moisturizers.

The present invention will be explained hereafter by means of a number of examples, whereby it should be noted, however, that the present invention is by no means restricted to such special examples.

### Example 1: Preparation of CaKPO₄ and Ca₂KNa(PO₄)₂.

An amount of 2 mole CaHPO₄ and 1 mole K₂CO₃ was thoroughly mixed and then heated at 1000 °C for one hour, and subsequently quenched in the air, after which the powder obtained as CaKPO₄ was ground to a fineness of less than 30 µm.

An amount of 4 mole CaHPO₄, 1 mole K₂CO₃ and 1 mole Na₂CO₃ was thoroughly mixed and then heated at 1000 °C for one hour, and subsequently quenched in the air, after which the powder obtained as Ca₂KNa(PO₄)₂ was ground to a fineness of less than 30 µm.

### Example 2: Spontaneous nucleation of K-A.

Part of the powder of CaKPO₄ as prepared in Example 1 above was mixed with 2 N acetic acid to form a pasta at pH 3.5. After a few hours the paste appeared to have been converted into K-A, which exhibited an X-ray diffraction pattern identical to the pattern of the nano apatite that occurs in bone and dentin. The same reaction occurs with a part of the powder of Ca₂KNa(PO₄)₂ as prepared in Example 1 above upon addition of 2 N acetic acid. When in both cases 2 N lactic acid at pH 2 was used in stead of 2 N acetic acid, the end products were also K-A with a nano apatite structure.

### Example 3.

The same procedure as disclosed in Example 2 was repeated, except that a paste was prepared at a pH value of 9. In that case the use of a calcium ion electrode showed that the equilibrium concentration of calcium is below 0.1 mmol which is too low for having good remineralization properties.

### Example 4: Stability of the Ca₂KNa(PO₄)₂ above pH 4.

An amount of 10 g Ca₂KNa(PO₄)₂ as prepared in Example 1 was mixed with 10 ml water, resulting in a pH of about 6-7 (Experiment A). Furthermore, 10 g Ca₂KNa(PO₄)₂ was mixed with 10 ml water, which contained 1% Na₂CO₃.10 H₂O and 1% KHCO₃ (resulting in a pH of about 8) (Experiment B). After 14 days of equilibration at room temperature, it appeared that neither the solid crystalline substance in Experiment A nor that in Experiment B had changed. This proves that Ca₂KNa(PO₄)₂ is indeed stable in a slightly acidic environment (Experiment A) and in a slightly alkaline environment (Experiment B).

## Claims

1. The use of CaKPO₄ and or Ca₂KNa(PO₄)₂ for the manufacture of a dental hygiene product which forms a precipitate of potassium containing apatite in the natural oral environment for providing remineralising power to the teeth, whereby the said hygiene product is buffered to a pH between 4 and 8.

2. The use of CaKPO₄ and/or Ca₂KNa(PO₄)₂ according to claim 1, **characterized in that** the amount of CaKPO₄ and/or Ca₂KNa(PO₄)₂ ranges between 1 and 80 wt.% based on the total weight of the hygiene product.

3. The use of CaKPO₄ and/or Ca₂KNa(PO₄)₂ according to claim 1, **characterized in that** the amount of CaKPO₄ and/or Ca₂KNa(PO₄)₂ ranges between 5 and 70 wt.% based on the total weight of the hygiene product.

4. The use of CaKPO₄ and/or Ca₂KNa(PO₄)₂ according to claim 1, **characterized in that** the amount of CaKPO₄ and/or Ca₂KNa(PO₄)₂ ranges between 20 and 50 wt.% based on the total weight of the hygiene product.

5. The use of CaKPO₄ and/or Ca₂KNa(PO₄)₂ according to claim 1, **characterized in that** a K⁺ and/or a Na⁺ containing buffer is used.

6. The use of CaKPO₄ and/or Ca₂KNa(PO₄)₂ according to any one of the preceding claims 1-5, **characterized in that** the hygiene product is a tooth paste.

7. The use of CaKPO₄ and/or Ca₂KNa(PO₄)₂ according to any one of the preceding claims 1-5, **characterized in that** the hygiene product is a chewing gum.

8. The use of CaKPO₄ and/or Ca₂KNa(PO₄)₂ according to any one of the preceding claims 1-5, **characterized in that** the hygiene product is a gel suspension.

9. The use of CaKPO₄ and/or Ca₂KNa(PO₄)₂ according to any one of the preceding claims 1-5, **characterized in that** the hygiene product is a mouth rinsing fluid.

10. The use of CaKPO₄ and/or Ca₂KNa(PO₄)₂ according to claim 9, **characterized in that** said mouth rinsing fluids are supersaturated with potassium containing apatite but are just saturated or slightly undersaturated with CaKPO₄ and/or Ca₂KNa(PO₄)₂ at the adjusted pH.

11. The use of CaKPO₄ and/or Ca₂KNa(PO₄)₂ according to any one of the preceding claims 1-5, **characterized in that** the hygiene product is a saliva substitute.

12. The use of CaKPO₄ and/or Ca₂KNa(PO₄)₂ according to claim 11, **characterized in that** said saliva substitutes are supersaturated with K-A, but just saturated or slightly undersaturated with CaKPO₄ and/or Ca₂KNa(PO₄)₂ at the adjusted pH.

13. The use of CaKPO₄ and/or Ca₂KNa(PO₄)₂ according to any of claims 9-12, **characterized in that** it comprises calcium ions, potassium ions and phosphate ions.

14. A dental hygiene product comprising CaKPO₄ and / or Ca₂KNa(PO₄)₂ which forms a precipitate of potassium containing apatite in the natural oral environment for providing remineralising power to the teeth, **characterised in that** said product, is buffered to a pH between 4 and 8.

## Patentansprüche

1. Verwendung von CaKPO₄ und/oder Ca₂KNa(PO₄)₂ zur Herstellung eines Zahnhygieneprodukts, das im natürlichen oralen Milieu einen Niederschlag von Kalium enthaltendem Apatit bildet, zur Remineralisierung der Zähne, wobei das Hygieneprodukt auf einen pH-Wert zwischen 4 und 8 gepuffert ist.

2. Verwendung von CaKPO₄ und/oder Ca₂KNa(PO₄)₂ nach Anspruch 1, **dadurch gekennzeichnet, dass** die Menge an CaKPO₄ und/oder Ca₂KNa(PO₄)₂ im Bereich zwischen 1 bis 80 Gew.-%, bezogen auf das Gesamtgewicht des Hygieneprodukts, liegt.

3. Verwendung von CaKPO₄ und/oder Ca₂KNa(PO₄)₂ nach Anspruch 1, **dadurch gekennzeichnet, dass** die Menge an CaKPO₄ und/oder Ca₂KNa(PO₄)₂ im Bereich zwischen 5 und 70 Gew.-%, bezogen auf das Gesamtgewicht des Hygieneprodukts, liegt.

4. Verwendung von CaKPO₄ und/oder Ca₂KNa(PO₄)₂ nach Anspruch 1, **dadurch gekennzeichnet, dass** die Menge an CaKPO₄ und/oder Ca₂KNa(PO₄)₂ im Bereich zwischen 20 und 50 Gew.-%, bezogen auf das Gesamtgewicht des Hygieneprodukts, liegt.

5. Verwendung von CaKPO₄ und/oder Ca₂KNa(PO₄)₂ nach Anspruch 1, **dadurch gekennzeichnet, dass** ein K⁺ und/oder Na⁺ enthaltender Puffer verwendet wird.

6. Verwendung von CaKPO₄ und/oder Ca₂KNa(PO₄)₂ nach einem der vorhergehenden Ansprüche 1 bis 5, **dadurch gekennzeichnet, dass** das Hygieneprodukt eine Zahnpasta ist.

7. Verwendung von CaKPO₄ und/oder Ca₂KNa(PO₄)₂ nach einem der vorhergehenden Ansprüche 1 bis 5, **dadurch gekennzeichnet, dass** das Hygieneprodukt ein Kaugummi ist.

8. Verwendung von CaKPO₄ und/oder Ca₂KNa(PO₄)₂ nach einem der vorhergehenden Ansprüche 1 bis 5, **dadurch gekennzeichnet, dass** das Hygieneprodukt eine Gelsuspension ist.

9. Verwendung von CaKPO₄ und/oder Ca₂KNa(PO₄)₂ nach einem der vorhergehenden Ansprüche 1 bis 5, **dadurch gekennzeichnet, dass** das Hygieneprodukt ein Mundspülmittel ist.

10. Verwendung von CaKPO₄ und/oder Ca₂KNa(PO₄)₂ nach Anspruch 9, **dadurch gekennzeichnet, dass** die Mundspülmittel beim eingestellten pH-Wert mit Kalium enthaltenden Apatit übersättigt sind, aber mit CaKPO₄ und/oder Ca₂KNa(PO₄)₂ gerade gesättigt oder schwach untergesättigt sind.

11. Verwendung von CaKPO₄ und/oder Ca₂KNa(PO₄)₂ nach einem der vorhergehenden Ansprüche 1 bis 5, **dadurch gekennzeichnet, dass** das Hygieneprodukt ein Speichelersatzprodukt ist.

12. Verwendung von CaKPO₄ und/oder Ca₂KNa(PO₄)₂ nach Anspruch 11, **dadurch gekennzeichnet, dass** die Speichelersatzprodukte beim eingestellten pH-Wert mit K-A übersättigt sind, aber mit CaKPO₄ und/oder Ca₂KNa(PO₄)₂ gerade gesättigt oder schwach untergesättigt sind.

13. Verwendung von CaKPO₄ und/oder Ca₂KNa(PO₄)₂ nach einem der Ansprüche 9 bis 12, **dadurch gekennzeichnet, dass** das Produkt Calciumionen, Kaliumionen und Phosphationen umfasst.

14. Zahnhygieneprodukt, umfassend CaKPO₄ und/oder Ca₂KNa(PO₄)₂, das im natürlichen oralen Milieu einen Niederschlag aus Kalium enthaltendem Apatit bildet, zur Remineralisierung der Zähne, **dadurch gekennzeichnet, dass** das Produkt auf einen pH-Wert zwischen 4 und 8 gepuffert ist.

## Revendications

1. Utilisation de CaKPO₄ et/ou de Ca₂KNa(PO₄)₂ pour la fabrication d'un produit d'hygiène dentaire pour former dans l'environnement buccal naturel, un précipité d'apatite contenant du potassium pour fournir une capacité de reminéralisation des dents, ledit produit d'hygiène étant tamponné à un pH compris entre 4 et 8.

2. Utilisation de CaKPO₄ et/ou de Ca₂KNa(PO₄)₂ selon la revendication 1, **caractérisée en ce que** la quantité de CaKPO₄ et/ou de Ca₂KNa(PO₄)₂ est de l'ordre de 1 à 80 % en poids par rapport au poids total du produit d'hygiène.

3. Utilisation de CaKPO₄ et/ou de Ca₂KNa(PO₄)₂ selon la revendication 1, **caractérisée en ce que** la quantité de CaKPO₄ et/ou de Ca₂KNa(PO₄)₂ est de l'ordre de 5 à 70 % en poids par rapport au poids total du produit d'hygiène.

4. Utilisation de CaKPO₄ et/ou de Ca₂KNa(PO₄)₂ selon la revendication 1, **caractérisée en ce que** la quantité de CaKPO₄ et/ou de Ca₂KNa(PO₄)₂ est de l'ordre de 20 à 50 % en poids par rapport au poids total du produit d'hygiène.

5. Utilisation de CaKPO₄ et/ou de Ca₂KNa(PO₄)₂ selon la revendication 1, **caractérisée en ce qu'**il est utilisé un tampon contenant K⁺ et/ou Na⁺.

6. Utilisation de CaKPO₄ et/ou de Ca₂KNa(PO₄)₂ selon l'une quelconque des revendications précédentes 1 à 5, **caractérisée en ce que** le produit d'hygiène est une pâte dentifrice.

7. Utilisation de CaKPO₄ et/ou de Ca₂KNa(PO₄)₂ selon l'une quelconque des revendications précédentes 1 à 5, **caractérisée en ce que** le produit d'hygiène est une gomme à mâcher.

8. Utilisation de CaKPO₄ et/ou de Ca₂KNa(PO₄)₂ selon l'une quelconque des revendications précédentes 1 à 5, **caractérisée en ce que** le produit d'hygiène est une suspension de gel.

9. Utilisation de CaKPO₄ et/ou de Ca₂KNa(PO₄)₂ selon l'une quelconque des revendications précédentes 1 à 5, **caractérisée en ce que** le produit d'hygiène est un bain de bouche liquide.

10. Utilisation de CaKPO₄ et/ou de Ca₂KNa(PO₄)₂ selon la revendication 9, **caractérisée en ce que** lesdits bains de bouche liquides sont sursaturés en apatite contenant du potassium, mais sont juste saturés ou légèrement sous-saturés en CaKPO₄ et/ou en Ca₂KNa(PO₄)₂ au pH ajusté.

11. Utilisation de CaKPO₄ et/ou de Ca₂KNa(PO₄)₂ selon l'une quelconque des revendications précédentes 1 à 5, **caractérisée en ce que** le produit d'hygiène est un substitut de la salive.

12. Utilisation de CaKPO₄ et/ou de Ca₂KNa(PO₄)₂ selon la revendication 11, **caractérisée en ce que** lesdits substituts de la salive sont sursaturés en K-A, mais juste saturés ou légèrement sous-saturés en CaKPO₄ et/ou en Ca₂KNa(PO₄)₂ au pH ajusté.

13. Utilisation de CaKPO₄ et/ou de Ca₂KNa(PO₄)₂ selon l'une quelconque des revendications 9 à 12, **caractérisée en ce qu'**ils comprennent des ions calcium, des ions potassium et des ions phosphate.

14. Produit d'hygiène dentaire comprenant du CaKPO₄ et/ou du Ca₂KNa(PO)₄ qui forme dans l'environnement buccal naturel, un précipité d'apatite contenant du potassium pour fournir une capacité de reminéralisation des dents, **caractérisé en ce que** ledit produit est tamponné à un pH compris entre 4 et 8.
